# EUROPEAN PATENT SPECIFICATION

(11) **EP 1 582 152 B1**
(45) Date of publication and mention of the grant of the patent: **06.04.2011**
(21) Application number: 04701686.0
(22) Date of filing: 13.01.2004
(51) Int. Cl.: A61B 17/12

(54) **EMBOLUS FORMING IN-VIVO INDWELLING IMPLANT**
EMBOLUS-BILDENDES IN-VIVO-VERWEILIMPLANTAT
IMPLANT A DEMEURE I IN VIVO /I FORMANT EMBOLE

(30) Priority: 10.01.2003 JP 2003005157
(43) Date of publication of application: 05.10.2005
(73) Proprietor: KANEKA CORPORATION, Osaka-shi, Osaka 5308228 (JP); Kaneka Medix Corporation, Osaka-shi, Osaka 5308228 (JP)
(72) Inventor: OGAWA, Atsushi, c/o KANEKA MEDIX CORPORATION, Kanagawa 2580113 (JP); SAKAI, Shinichi, c/o KANEKA MEDIX CORPORATION, Kanagawa 2580113 (JP)
(74) Representative: Gille Hrabal Struck Neidlein Prop Roos
(86) International application number: PCT/JP2004/000135
(87) International publication number: WO 2004/062509

(56) References cited:
- EP-A1- 0 792 623
- WO-A-2004/002329
- WO-A1-98/58590
- WO-A1-99/09894

## Description

### TECHNICAL FIELD

The present invention relates to an indwelling implant for embolization, more particularly to an indwelling implant for embolization suitable for modifying or interrupting blood flow and for embolizing pathology sites.

### BACKGROUND ART

Reference information on the prior art technology relating to the invention of the present application is described in Japanese Patents Nos. 3023076 and 2909021, Japanese Patent Application Laid-open No. H8-187248, and Japanese Patent Publication No. 2002-507902.

Vascular embolization by which an indwelling implant for embolization is indwelled in an aneurysm is presently known as a low invasive method for treating aneurysms and the like. With this vascular embolization method, an indwelling implant for embolization that was indwelled inside an aneurysm becomes a physical obstacle for a blood flow and also can reduce the risk of aneurysm rupture by forming thrombi around the indwelling implant for embolization.

Such indwelling implant for embolization is used, for example, by introducing it to a prescribed site via an appropriate catheter with push-out means (delivery device) detachably connected to the end portion of the indwelling implant for embolizing and detaching and indwelling it in the prescribed site.

Such an indwelling implant for embolization is required to have the following characteristics.
(1) It is required to have high flexibility which is necessary to conduct the indwelling operation without problems such as puncture of the blood vessel or aneurysm wall surface caused by application of excess load to the utilization site such as the blood vessel or aneurysm.
   Further, it is also necessary for the indwelling implant for embolization to have high flexibility so as to maximize the embolization ratio per unit volume (the occupation ratio of the indwelling implant for embolization in the prescribed site) and to enable easy insertion even in tiny gaps of the utilization site during indwelling operation in order to prevent the occurrence of gaps caused by changes in shape with time and also to prevent the occurrence of recanalization (coil compaction).
(2) The indwelling implant for embolization is also required to have a function of preventing or inhibiting the unlimited extension of the coil and thereby making it possible to conduct reliably the re-indwelling operation of retrieving the indwelling implant and correcting the position thereof after it has been pushed out of the catheter and disposed in the prescribed site. Thus, the indwelling implant is required to have a configuration eliminating the problems associated with the difficulty of recovering the coil caused by the extension of the coil that was caught, for example, at the distal edge of the catheter, or with a risk of damaging the utilization site.
(3) A method of fixing the extension controlling member provided in the axial direction inside the coil at both ends thereof is employed as means for preventing or inhibiting the extension of the coil. For the reasons stated hereinabove, the extension controlling member is required to have a high strength and also to be flexible. Thus, a metal material with a strength higher than that of other materials is preferably used for the extension controlling member in order to further increase safety during operation. However, in addition to providing the necessary strength, it is also necessary that flexibility be ensured by using a wire material with as small a thickness as possible, so that flexibility of the coil is not lost.
(4) A material safe to a living body has to be used. From the standpoint of safety to a living body, it is preferred that the coil and the extension controlling member be produced from a metal material which demonstrates stability in long-term indwelling in a living body higher than that of other materials. Furthermore, a welding method is preferred as the fixing method for the coil and extension controlling member in order to avoid the addition of materials of other types such as adhesives or solders.
   However, when the extension controlling member was welded to the coil, the strength of the extension controlling member in the welded zone was greatly reduced by annealing. Therefore, the cross sectional area of the extension controlling member had to be sufficiently increased in order to obtain a strength necessary for the extension controlling member. The resultant problem was that flexibility of the indwelling implant for embolization was lost.
   WO2004/002329 is prior art in the meaning of Article 54(3) EPC and discuses an indwelling embolisation coil with an extension controlling member. No loop is disclosed as being welded to the head portion.
   WO99/09894 is considered to represent the closest prior art in the meaning of Article 54 (1,2) EPC) and Article 56 EPC and discloses an indwelling embolisation coil in which a thinner stretch resisting wire (eg metallic or polymer) is attached to a thicker loop at a proximal end of the coil. WO89/09894 is silent as to the nature of the other end. WO98/58590 discloses an indwelling embolisation call in which a stretch resisting wire extends between two rounded head portions.

### DISCLOSURE OF THE INVENTION

The present invention was created with the foregoing in view and it is an object thereof to provide an indwelling implant for embolization which has high flexibility required to introduce and indwell it in the prescribed site in a body and makes it possible to execute safely the re-indwelling operation of the indwelling implant by preventing or inhibiting the unraveling of the coil, for example, even when a re-indwelling operation is conducted, for example, by recovering the indwelling implant and correcting the position thereof after it has been disposed in the body, thereby providing for high safety and high operability.

An indwelling implant for embolization in accordance with the present invention is set out in appended claim 1.

In the indwelling implant for embolization in accordance with the present invention, the axial extension controlling member is preferably composed of the same metal material as the coil, and the coil or axial extension controlling member is preferably composed of a platinum alloy. Further, the axial extension controlling member is preferably composed of a twisted wire obtained by twisting together a plurality of metal wire materials.

Further, the indwelling implant for embolization in accordance with the present invention can have a configuration which is obtained by secondary molding such as further spirally winding the coil and forming a secondary coil.

Further, in the indwelling implant for embolization in accordance with the present invention, the loop may be provided only at the proximal end portion or both at the distal end portion and proximal end portion, rather than only at the distal end portion of the coil.

Because a metal material is used as the axial extension controlling member used in the indwelling implant for embolization in accordance with the present invention, a loop is formed from a wire material which is thicker than the axial extension controlling member at the distal end portion of the coil, and the axial extension controlling member is fixed by passing through the loop and hanging thereon, the reduction in strength of the welded zone of the axial extension controlling member which is caused by annealing is prevented. Therefore, a wire material providing for a necessary strength and having a sufficiently small thickness can be used as the axial extension controlling member and the indwelling implant can have a configuration with high flexibility. Therefore, high operability can be obtained during indwelling operation and, for example, the indwelling implant can be reliably introduced and indwelled in the prescribed site via an appropriate catheter.

Furthermore, when a strong impact is applied instantaneously as a stress in the axial direction of the coil, because the axial extension controlling member is fixed to the distal end portion of the coil via the loop, a sufficient dimensional margin is produced by deflection in the crossing zone of the loop and the axial extension inhibiting member. Therefore, a configuration can be obtained in which the impact is absorbed by this dimensional margin. As a result, a contribution can be made to the increase in impact resistance of the coil as an extension preventing mechanism. Furthermore, when the axial extension controlling member is composed of a twisted wire obtained by twisting together a plurality of metal wire materials, the twisted wire itself has impact resistance. Therefore, the impact resistance of the coil as an extension preventing mechanism can be further increased.

On the other hand, composing the axial extension controlling member of a metal identical to that of the coil facilitates fixing by welding, can prevent the occurrence of galvanic corrosion induced by contact between different metal materials in the environment inside a living body, and makes it possible to obtain a configuration in which safety with respect to a living body during long-term indwelling is further increased. Furthermore, composing the coil or the axial extension controlling member of a metal stable in a living body, such as a platinum alloy, can further increase safety with respect to a living body during long-term indwelling.

The above-described operation is the same when the loop is provided at the proximal end portion or both at the distal end portion and proximal end portion, rather than only at the distal end portion of the coil.

### BRIEF DESCRIPTION OF THE DRAWINGS

FIG. 1 is a cross-sectional view illustrating schematically the configuration which is an example of the indwelling implant for embolization in accordance with the present invention; and
FIG. 2 is a cross-sectional view illustrating schematically the configuration which is another example of the indwelling implant for embolization in accordance with the present invention

### BEST MODE FOR CARRYING OUT THE INVENTION

The present invention will be described hereinbelow in greater detail with reference to the appended drawings.

### <Embodiments>

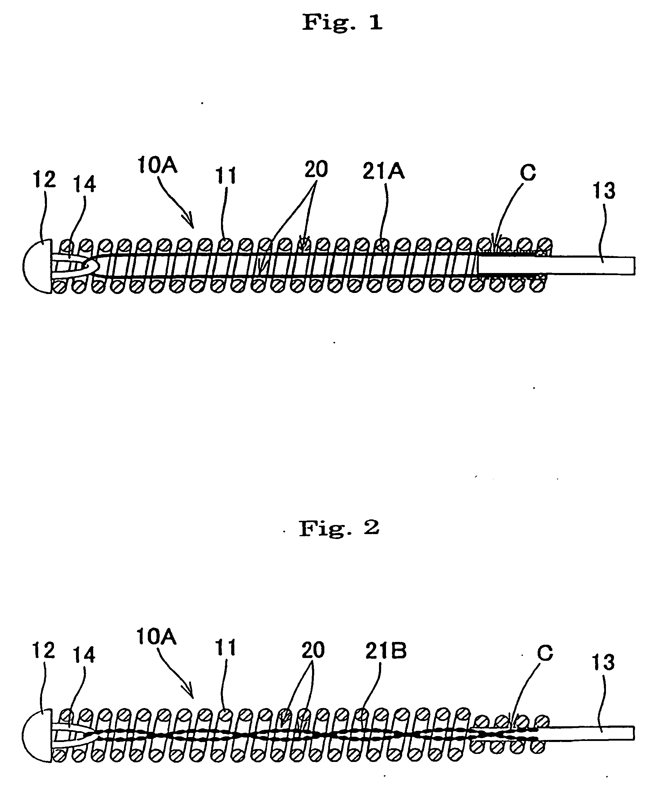

FIG. 1 is an explanatory cross-sectional view illustrating schematically the configuration representing an example of the indwelling implant for embolization in accordance with the present invention.

This indwelling implant for embolization (simply referred to hereinbelow as "indwelling implant") 10A comprises a metal coil 11 which is preferably flexible. A hemispherical rounded head portion 12 is provided in the distal end portion (left end portion in FIG. 1) of the metal coil 11. Furthermore, for example, a rod-like joint member 13 for supporting the metal coil 11 is so provided in the base end portion of the metal coil 11 that it protrudes and extends outwardly (to the right in FIG. 1) in the axial direction of the coil from the edge of the base end of the metal coil 11 after part thereof has been fixed to the inner peripheral surface of the based end portion of metal coil 11. Further, in the indwelling implant for h embolization of the present application, the hemispherical rounded head portion 12 can be of other shape, provided it does not damage the blood vessels.

The joint member 13 is so provided that the metal coil 11 can be connected to an appropriate delivery wire or catheter or detached therefrom.

The metal coil 11 constituting the indwelling implant 10A is generally composed by spirally winding a metal wire material. The metal wire material can be selected from materials that are chemically stable, like noble metals, with respect to a human body when they are retained for a long time therein, are chemically stabilized via the formation of a passivation film on the surface in a living body, and have low toxicity (good biocompatibility). Examples of such materials include platinum, gold, titanium, tungsten, alloys thereof, stainless steel and the like. It is preferred that a platinum alloy such as a platinum tungsten alloy be used because such an alloy combines good processability and good physical properties such as strength and elasticity with chemical stability in a living body.

As described hereinabove, the metal coil 11 constituting the indwelling implant 10A preferably has flexibility or bendability and preferably has the following configuration which may differ depending on the material of the metal wire constituting the metal coil 11.

For example, the diameter (primary wire diameter) of the metal wire material constituting the metal coil 11 is 10-120 µm, the coil diameter of the metal coil 11 is 100-500 µm, and the coil length is 2-500 mm.

In such an indwelling implant 10A, a metal loop 14 is welded to the substantially semispherical head 12 inside (inside a tubular body) of the metal coil 11, this loop being directed toward the proximal end of the metal coil 11. An axial extension controlling member 20 composed of a metal wire material composed, for example, of a single metal wire 21A is passed through the metal loop 14. One end and the other end of the axial extension controlling member are fixed to the proximal end portions of the metal coil 11, and the entire axial extension controlling member extends in the coil axial direction of the metal coil 11. As a result, the metal coil 11 is so provided that it is free to extend within a range allowed by the tension of the axial extension controlling member 20. Here, in FIG. 1, the location shown by hatching with a grid-like pattern (shown by an arrow C) is a fixing location of the metal wire 21A. In the indwelling implant for embolization of the present application, the metal loop 14 and axial extension controlling member 20 can be formed from a polymer, but forming them from a metal is preferred from the standpoint of safety (chemical stability inside a living body and physical properties such as strength and elasticity).

The metal wire 21A constituting the metal loop 14 and axial extension controlling member 20 have a tensile rupture strength larger than at least the minimum stress required for permanent deformation of the metal coil 11 by extension in the axial direction of the coil. The metal wire 21A is composed of a material in which the cross section area of the wire with a high tensile rupture strength inherent to the metal wire material constituting the metal wire 21A is made as small as possible, so that the axial extension controlling member 20 has an anticipated strength, to prevent reliably the metal coil 11 from actually extending limitlessly in the axial direction of the coil and to avoid the degradation of flexibility of the metal wire 11 itself.

More specifically, the metal wire 21A and metal loop 14 are preferably composed of a material in which the tensile rupture strength inherent to the metal wire material constituting the metal wire 21A is, for example, 700 N/mm² or more, more preferably 900-5000 N/mm². Specific examples of such metal wire materials include platinum, gold, titanium, tungsten, alloys thereof, and stainless steel. Using a platinum alloy such as a platinum-tungsten alloy is preferred because such an alloy combines good processability and good physical properties such as strength and elasticity with chemical stability in a living body. Furthermore, composing it of the same metal material as the metal coil 11, for example, a platinum alloy such as a platinum-tungsten alloy, is preferred because the occurrence of galvanic corrosion caused by contact of different metal materials in the environment inside a living body can be avoided and safety with respect to a living body in long-term indwelling can be further increased.

The metal wire 21A is preferably composed of a wire material such as a round or angular wire with a diameter (wire diameter) of, for example, 25 µm or less, more preferably 5-20 µm, or a flat wire with a thickness of 2-20 µm. Furthermore, using a twisted wire which is obtained by twisting such metal wires is preferred because the structure of the twisted wire itself has impact resistance and the impact resistance of the coil as an extension preventing mechanism can be increased. It is also preferred that the impact resistance be further increased with a structure obtained by additional twisting after the metal wire 21A has been passed through the metal loop 14.

The metal wire material constituting the metal loop 14 may have a large size such that the formed loop can be disposed inside the metal coil 11 and also can have a strength somewhat higher than the axial tensile rupture strength of the metal wire 21A even when the strength was decreased by annealing caused by heat during welding of the head 12 (this strength is difficult to adjust, but the identical strength can be obtained). It is also preferred than the metal loop be composed of a wire material, such as a round wire, angular wire, or a flat wire with a large cross section area, which is thicker than the wire material constituting the metal wire 21A. In the indwelling implant for embolization of the present application, a wire material with an axial tensile rupture strength higher than the axial tensile rupture strength of the metal wire 21A has to be used as the metal wire material constituting the metal loop 14. To attain this object, the composition of the metal wire 21A has to be so changed as to obtain a high strength. Another option is to form it from a material with a diameter equal to or less than that of the metal wire 21A. However, it is preferred that in this case, measures be taken to prevent the formation of galvanic electrodes or that the implant be used only for applications where such galvanic electrodes cause no problem.

Further, when the metal wire 21A is tensioned after the metal wire 21A it has been passed through the loop, if the cross-sectional structure of the metal loop 14 has an acute angle, then a stress concentration point is provided for a rupture process of the metal wire 21A and rupture strength is somewhat decreased. Therefore, an elliptical or perfectly round wire with a cross section having no portions with acute angle is preferred over an angular wire. Furthermore, no specific limitation is placed on the length of the metal loop 14, but it is preferred that the length of the loop be as small as possible by comparison with the length of the metal coil, so as to obtain the expected flexibility of the metal coil 11.

As for the axial extension controlling member 20 in the indwelling implant 10A, the tensile rupture strength of the entire axial extension controlling member 20 with a specified configuration, such as the material type and wire diameter of the metal coil 21A, is preferably 0.1 N or more, more preferably 0.2-1 N. The " tensile rupture strength [N]" as referred to herein is, for example, a value inherent to the axial extension controlling member 20 with a specified configuration, such as the material type and wire diameter of the metal coil 21A, and can be represented by a maximum load necessary to rupture such specific axial extension controlling member 20. The tensile strength of the entire axial extension controlling member 20 in the indwelling implant 10A shown in FIG. 1 is substantially equivalent to about twofold tensile rupture strength inherent to the metal wire material constituting the metal wire 21A.

Welding can be used as means for fixing the metal loop 14 to the substantially hemispherical rounded head 12, but no specific limitation is placed on the welding means. For example, generally employed methods such as resistance welding, MIG, TIG, laser welding, and those methods conducted in an inert gas atmosphere can be employed.

No specific limitation is placed on means for fixing both ends of the metal wire 21A to the proximal end portion of the metal coil 11. For example, adhesive bonding with an adhesive, welding or fusion via an insert, press joining such as mechanical caulking, physical connection, ligatures and other means can be used for this purpose.

An embodiment in which the metal wire 21A was used as the material constituting he axial extension controlling member 20 was described above. However, the axial extension controlling member 20 can have various configurations provided that it has a tensile rupture strength higher than a minimum stress required for the metal coil 11 to be deformed permanently by extension in the axial direction of the coil and that the flexibility of the metal coil 11 itself is not degraded.

For example, as shown in FIG. 2, the axial extension controlling member 20 can be composed of a twisted wire 21B formed by gently twisting a plurality of metal wires together and can have a structure in which the metal wire 21B is further twisted after being passed through the metal loop 14. Materials that were presented as examples of materials constituting the metal wire 21A in the indwelling implant 10A shown in FIG. 1 can be used as the materials for constituting each metal wire mentioned hereinabove.

In this case, if each of the metal wires constituting the twisted wire 21B uses a metal wire material identical to that of the metal wire 21A in the indwelling implant 10A shown in FIG. 1, the diameter (unit wire diameter) thereof can be less than that of the metal wire 21A of the indwelling implant 10A shown in FIG. 1.

More specifically, the value of the diameter of each metal wire differs depending on the number and material of the metal wires which are to be twisted, but it can be appropriately selected so that the tensile rupture strength of the entire twisted wire 21B is, for example, 0.1 N or more.

The aforementioned indwelling implant 10A can be used in the above-described linear primary form, but from the standpoint of increasing operability or embolizing performance in the prescribed location, it is preferred that it be used in a form in which a secondary coil is formed by further spirally winding a coil composed of the linear metal coil 11, for example, in a form obtained by forming into an S-shaped, J-shaped, three-dimensional structure, or other secondary form.

A method for using the above-described indwelling implant 10A will be described hereinbelow with reference to an example of employing it for treating an aneurysm.

First, an appropriate catheter is percutaneously inserted into a blood vessel by using a puncturing needle and so disposed that the distal end of the catheter reaches the aneurysm inlet. The indwelling implant 10A shaped in advance to assume a secondary form, for example, such as a secondary coil is detachably attached to a distal end of a guide wire. Then, in a state in which the indwelling implant 10A was linearly stretched and returned to a primary form (the form shown in FIG. 1 and FIG. 2), the guide wire is inserted into the catheter and advanced therein. The indwelling implant 10A is then pushed out through the distal end of the catheter and disposed inside the aneurysm.

Here, the following methods can be used for detaching the indwelling implant 10A from the guide wire: (1) a method employing a structure for mechanically engaging and mechanically detaching the guide wire and the joint member 13 in the indwelling implant 10A, and (2) a method employing a structure using current-induced electrolytic separation such that, for example, a monopolar highfrequency electric current is supplied and the joint member 13 in the indwelling implant 10A is heated with the highfrequency current, melted, and broken thereby providing for the separation from the guide wire.

If the indwelling implant 10A is pushed out of the catheter, it restores the secondary coil shape and assumes a three-dimensionally intertwined configuration. In this state, the complete insertion of the indwelling implant 10A into the aneurysm is confirmed by fluoroscopy and then the indwelling implant 10A is separated from the distal end of the guide wire and left in place.

If necessary, a plurality of indwelling implants 10A are used and the above-described operations are repeated to fill the inside of the aneurysm with a plurality of indwelling implants 10A and form a blood plug, thereby preventing the flow of blood into the aneurysm and thus preventing the aneurysm reliably from rupture.

With the indwelling implant 10A in accordance with the present invention, the metal loop 14 composed of a wire material thicker than the axial extension controlling member 20 is welded to the head 12 disposed in the distal end portion of the metal coil 11 and the metal wire 21A constituting the axial extension controlling member 20 is inserted and pulled through the loop 14, thereby fixing the axial extension controlling member 20 to the distal end portion of the metal coil 11. As a result, the annealing-induced decrease in strength of the welded portion which occurred when the axial extension controlling member 20 was directly welded to the distal end portion of the metal coil 11 is prevented. Therefore, a strength level necessary for the stretching preventing mechanism can be obtained. Moreover, a sufficiently thin wire material can be used and a configuration with a highly flexible indwelling implant can be obtained. Therefore, high operability can be obtained when an indwelling operation is carried out and indwelling can be conducted, for example, by reliably introducing the implant to the prescribed site via an appropriate catheter.

Moreover, in such an indwelling operation, it is sometimes necessary to recover the indwelling implant 10A that has been disposed by pulling it back inside a catheter and repeating the indwelling operation in order to dispose the indwelling implant 10A in an accurate position in the prescribed site. With the indwelling implant 10A in accordance with the present invention, in order to obtain the metal wire 21A such that the metal coil 11 is actually reliably prevented from limitlessly elongating in the axial direction of the coil and flexibility degradation of the metal coil 11 itself is prevented, the metal wire material constituting the metal wire 21A which has a high inherent tensile rupture strength is composed of a wire such that the cross sectional area thereof is decreased as much as possible so that the axial extension controlling member 20 has the anticipated strength. Therefore, the metal wire has the initial strength and flexibility. As a result, the re-indwelling operation comprising a step of recovering the indwelling implant 10A can be conducted reliably and an indwelling implant with a high safety can be obtained.

Furthermore, when a strong impact is instantaneously applied as a stress in the axial direction of the metal coil 11, because the axial extension controlling member 20 is fixed to the distal end portion of the metal coil 11 via the metal loop 14, a certain dimensional margin is created by the deflection of the axial extension controlling member 20 with respect to the entire length of the metal coil 11. Therefore, a configuration can be obtained in which the impact is absorbed by this dimensional margin. As a result, a contribution can be made to the improvement of impact resistance of the metal coil 11 as an extension preventing mechanism.

Furthermore, as shown in FIG. 2, when the axial extension controlling member 20 is composed of a twisted wire which is obtained by twisting together a plurality of metal wire materials, the twisted wire structure itself has impact resistance. Therefore, the impact resistance of the coil as the extension preventing mechanism can be additionally further increased. Moreover, a structure can be obtained in which the metal wire 21A is further twisted after being inserted through the metal loop 14.

On the other hand, forming the axial extension controlling member 20 from a metal identical to that of the metal coil 11 facilitates fixing by welding, can prevent the occurrence of galvanic corrosion caused by contact between metal materials of different types in the environment inside a living body, and can further increase safety with respect to the living body during long-term indwelling. Moreover, producing the metal coil 11 or axial extension controlling member 20 from a metal which is stable in a living body such as a platinum metal, allows safety with respect to the living body during long-term indwelling to be further increased.

The preferred embodiment of the present invention was explained above, but the present invention is not limited to the above-described embodiment, and in the indwelling implant 10A with the configuration shown in FIG. 1, the axial extension controlling member 20 can be formed as a coil by spirally winding a metal wire or from a metal wire that was formed into a zigzag or ribbon-like shape.

In this case, the coil diameter, coil length, and other structural features of the coil body which is to be formed from the metal wire may be so set that the elongation of the metal coil 11 in the axial direction of the coil is controlled to a fixed value or below and the indwelling implant has the prescribed flexibility.

### <Experimentation Examples>

Examples of tests carried out to confirm the operation effect of the indwelling implant in accordance with the present invention will be described below.

### <Manufacturing Example 1>

A metal loop with an elemental wire diameter of 30 µm and a total length of 2 mm that was formed of a platinum - tungsten alloy wire material was inserted toward a proximal end into a distal end portion of a metal coil (coil diameter 250 µm, coil length 30 mm) formed from the same platinum - tungsten alloy wire material with an elemental wire diameter of 40 µm, and the coil end and the metal loop were welded by TIG welding under an argon-helium mixed gas with the object of preventing oxidation. At this time, as a result of welding the metal loop to the distal end portion of the metal coil, the hemispherical rounded head portion was naturally formed at the distal end of the metal coil under the effect of surface tension of the metal. A platinum - tungsten alloy wire with an elemental wire diameter of 12 µm and a total length of 60 mm was inserted as an axial extension controlling member into the metal loop, both ends of the platinum - tungsten alloy wire were introduced between a holding member and the inner surface of the metal coil, the gap therebetween was filled with an adhesive, thereby fixing the metal coil and the platinum - tungsten alloy wire and producing an indwelling implant shown in FIG. 1 in which the platinum - tungsten alloy wire was fixed at the distal end of the coil with the loop. This indwelling implant will be called "indwelling implant

### <Manufacturing Example 2>

In the Manufacturing Example 1, the platinum - tungsten alloy of the materials forming the coil, metal loop and axial extension controlling member was replaced with a platinum - iridium alloy, the axial extension controlling member was composed of a twisted wire obtained by loosely twisting three platinum - iridium alloy wires with an elemental wire diameter of 7 µm and a total length of 60 mm, after the axial extension controlling member has been inserted through the metal loop, the wire was further twisted, and then both ends of the axial extension controlling member were caulked together with the joint member 13 to shrink and join them to a proximal end portion of the coil. Other features were identical to those of the Manufacturing Example 1. As a result, an indwelling implant with the configuration shown in FIG. 2 was obtained.
This indwelling implant will be called "indwelling implant 2".

### <Comparative Manufacturing Example 1>

The axial extension controlling member was composed of a platinum - tungsten alloy with an elemental wire diameter of 12 µm and a total length (30 mm) same as the coil length of the metal coil in the usual state. One end of the axial extension controlling member was directly welded to the distal end portion of the coil. The other end was introduced between the inner surface of the holding member and metal coil, and the gap therebetween was filled with adhesive, thereby fixing this other end to the metal coil. An indwelling implant was thus manufactured, other features being the same as in the Manufacturing Example 1. This indwelling implant be called "comparative indwelling implant 1".

### <Comparative Manufacturing Example 2>

The axial extension controlling member was composed of a platinum - tungsten alloy with an elemental wire diameter of 30 µm and a total length (30 mm) same as the coil length of the metal coil in the usual state. One end of the axial extension controlling member was directly welded to the distal end portion of the coil. The other end was introduced between the inner surface of the holding member and metal coil, and the gap therebetween was filled with an adhesive, thereby fixing this other end to the metal coil. An indwelling implant was thus manufactured, other features being the same as in the Manufacturing Example 1. This indwelling implant be called "comparative indwelling implant 2".

### <Comparative Manufacturing Example 3>

An indwelling implant was manufactured in the same manner as in the Manufacturing Example 1 , except that the metal loop and axial extension controlling member were not provided and only the coil was attached to the holding member. This indwelling implant be called "comparative indwelling implant 3".

With respect to the indwelling implants 1 and 2 and comparative indwelling implants 1 to 3 manufactured in the above-described manner, the following properties were evaluated: (1) the maximum load required for plastic deformation in the axial direction of the coil, this load being equal to a tensile rupture strength of the axial extension controlling member in the axial direction of the coil (tensile rupture strength in the member of specific configuration), and (2) flexibility of the entire indwelling implant. The results are shown in Table 1 below.

The tensile rupture strength of the axial extension controlling member was obtained by using a tension-compression test machine "Strograph E-L (manufactured by Toyo Precision Machinery Co.) and conducting a tension test by chucking both ends of the metal coil in the indwelling implant under normal-temperature environment and employing a load cell scale 2.5 NFS and a tension rate of 100 mm/min.

Further, flexibility of the entire indwelling implant was evaluated by comparing manual feeling and the degree of sagging when one end of the indwelling implant was grasped (based on external appearance) with those of the comparative indwelling implant 3.

**Table 1. Permanent deformation stress of indwelling implant and flexibility of the entire ii**

| | Tensile rupture strength of axial extension controlling member (N) | Flexibility of the entire indwelling implant (manual feeling and external appearance) |
|---|---|---|
| Indwelling implant 1 (present invention) | 0.64 | Very flexible, same as in comparative indwelling implant 3 |
| Indwelling implant 2 (present invention) | 0.62 | Very flexible, same as in comparative indwelling implant 3 |
| Comparative indwelling implant 1 | 0.08 | Very flexible, same as in comparative indwelling implant 3 |
| Comparative indwelling implant 2 | 0.60 | Harder than in comparative indwelling implant 3, unsuitable for use |
| Comparative indwelling implant 3 | 0.07 | Very flexible |

| | | |
|---|---|---|
| * Permanent deformation stress (yield stress) | | |

The results presented hereinabove confirmed that with the indwelling implant in accordance with the present invention (indwelling implants 1 and 2), the axial extension controlling member can be constructed as a member having a sufficiently high tensile rupture strength, extension of the metal coil in the axial direction of the coil can be thereby controlled, the indwelling implant can be constructed as an implant with high flexibility, and therefore excellent operability and high safety during indwelling operation can be obtained.

Here, if the elongation of the metal coil (coil) does not exceed a yield point, the metal coil can be deformed within an elastic region. Therefore, the original state can be restored by releasing the load applied to the metal coil. It is desired that the metal coil have as low an elongation as possible when the indwelling operation is conducted, but without loosing the characteristics of the metal coil in practical use.

By contrast, in the comparative indwelling implant 1, the indwelling implant has a sufficient flexibility, but the strength is reduced because the axial extension controlling member is directly welded to the coil and the desired strength cannot not be obtained. As a result, there is supposedly a high probability of the coil stretching during indwelling operation of the indwelling implant.

Furthermore, in the comparative indwelling implant 2, a wire material with a large diameter is used for the axial extension controlling member to compensate for the reduction in strength during direct welding of the axial extension controlling member. Therefore, the axial extension controlling member has a sufficiently high tensile rupture strength and the metal coil demonstrates substantially no extension in the axial direction of the coil. However, the indwelling implant as a whole has a very low flexibility and it can be supposed that the indwelling operation of the indwelling implant will be sometimes difficult to conduct reliably.

### INDUSTRIAL APPLICABILITY

With the indwelling implant for embolization in accordance with the present invention, a loop from a wire material with a thickness larger than that of an axial extension controlling member is formed in the distal end portion of a coil and the axial extension controlling member is fixed by passing through the loop and hanging thereon. As a result, the reduction in strength caused by annealing of the welded portion of the axial extension controlling member that occurs in case of direct welding is avoided. Therefore, a strength necessary for the axial extension controlling member can be obtained and a wire material of a sufficiently small diameter can be used. As a result, the indwelling implant can be constructed as an implant with high flexibility. Therefore, high operability during indwelling operation can be obtained. For example, the indwelling implant can be reliably inserted to the prescribed site and indwelled via an appropriate catheter.

Furthermore, when a strong impact is instantaneously applied as a stress acting in the axial direction of the coil, because the axial extension controlling member is fixed via the loop in the distal end portion of the coil, a certain dimensional margin is created by the deflection of the axial extension controlling member of the axial extension controlling member with respect to the entire length of the coil. Therefore a configuration can be obtained in which impacts are absorbed by this dimensional margin. As a result, a contribution can be made to the improvement of impact resistance of the coil as an extension preventing mechanism. Furthermore, when the axial extension controlling member is composed of a twisted wire obtained by twisting a plurality of metal wires, because the twisted wire structure itself has impact resistance, the impact resistance of the coil as an extension preventing mechanism can be additionally increased.

On the other hand, composing the axial extension controlling member of a metal identical to that of the coil facilitates fixing by welding, can prevent the occurrence of galvanic corrosion induced by contact between different metal materials in the environment inside a living body, and makes it possible to obtain a configuration in which safety with respect to a living body during long-term indwelling is further increased. Furthermore, composing the coil or the axial extension controlling member of a metal stable in a living body, such as a platinum alloy, can further increase safety with respect to a living body during long-term indwelling.

## Claims

1. An indwelling implant (101) for embolization comprising a coil (11) composed of a metal and extending from a substantially hemi-spherical rounded head portion is situated at the distal end portion of the coil, to a proximal portion of the coil, wherein a closed metal loop (14) is provided inside said coil so as to protrude from said head portion toward the proximal end portion of the coil, whereby the loop is welded to the semi-spherical head portion
and an axial extension controlling member (20) composed of at least one wire material which is thinner than the metal wire material forming said loop is provided inside said coil in such manner that the axial extension controlling member extends in the coil axial direction of said coil and passes through said loop, and both ends of the wire material forming the axial extension controlling member are directly or indirectly fixed to the proximal end portion.

2. The indwelling implant for embolization according to claim 1, wherein the axial extension controlling member and loop are composed of the same metal material as the coil.

3. The indwelling implant for embolization according to claim 1 or 2, wherein the coil is composed of platinum or a platinum alloy.

4. The indwelling implant for embolization according to any claim of claims 1 to 3,
wherein the axial extension controlling member is composed of a wire material with a diameter of 20 µm or less.

5. The indwelling implant for embolization according to any claim of claims 1 to 4,
wherein the axial extension controlling member is composed of a twisted wire obtained by twisting together a plurality of metal wire materials.

6. The indwelling implant for embolization according to any claim of claims 1 to 5,
wherein the axial extension controlling member is further twisted after insertion through the loop.

7. The indwelling implant for embolization according to any claim of claims 1 to 6,
wherein the coil is further formed to have a secondary shape.

8. The indwelling implant for embolization according to any one of claims 1 to 7,
wherein the head portion is formed under the effect of surface tension of the metal when welding the loop to the distal end portion of the metal coil.

9. A method for manufacturing the indwelling implant for embolization of claim 8. comprising steps of inserting the loop into the distal end portion of the coil toward the proximal direction, and forming the head portion by a metal surface tension when wielding the loop.

## Patentansprüche

1. Ein Dauerimplantat (10A) zur Embolisierung, umfassend einen Bandring (11), welcher aus einem Metall zusammengesetzt ist und sich von einem im Wesentlichen halbkugelförmigen Kopfteil (12), welches sich an dem Distalendteil des Bandringes befindet, zu einem rumpfförmigen Teil des Bandringes erstreckt, worin eine geschlossene metallhaltige Schlinge (14) innerhalb des Bandringes so vorgesehen ist, dass diese sich von dem Kopfteil in Richtung des rumpfförmigen Endteils des Bandringes erstreckt, wobei die Schlinge mit dem halbkugelförmigen Kopfteil verschweißt ist, und worin
ein Element (20), welches die axiale Ausrichtung steuert und welches zusammengesetzt ist aus mindestens einem Kabelmaterial, welches dünner ist als das Metallkabelmaterial, welches die Schlinge bildet, innerhalb des Bandringes so vorgesehen ist, dass das Element, welches die axiale Ausrichtung steuert, sich in die axiale Richtung des Bandringes erstreckt und durch die Schlinge geführt wird und beide Enden des Kabelmaterials, welches das Element, welches die axiale Ausrichtung steuert, bildet, unmittelbar oder mittelbar an dem rumpfähnlichen Endteil fixiert sind.

2. Das Dauerimplantat zur Embolisierung nach Anspruch 1 , worin das Element, welches die axiale Ausrichtung steuert und die Schlinge aus demselben Metallmaterial wie der Bandring zusammengesetzt sind.

3. Das Dauerimplantat zur Embolisierung gemäß Anspruch 1 oder 2, worin der Bandring aus Platin oder einer Platinlegierung besteht.

4. Das Dauerimplantat zur Embolisierung nach einem der Ansprüche 1 bis 3, worin das Element, welches die axiale Ausrichtung steuert, zusammengesetzt ist aus einem Kabelmaterial mit einem Durchmesser von 20 µm oder weniger.

5. Das Dauerimplantat zur Embolisierung nach einem der Ansprüche 1 bis 4, worin das Element, welches die axiale Ausrichtung steuert, zusammengesetzt ist aus einem verdrillten Kabel, welches erhalten wird durch gemeinsames Verdrillen einer Mehrzahl von Metallkabelmaterialien.

6. Das Dauerimplantat zur Embolisierung nach einem der Ansprüche 1 bis 5, worin das Element, welches die axiale Ausrichtung steuert, darüber hinaus nach dem Einführen in die Schlinge verdrillt wird.

7. Das Dauerimplantat zur Embolisierung nach einem der Ansprüche 1 bis 6, worin der Bandring darüber hinaus so ausgebildet ist, dass er eine sekundäre Form aufweist.

8. Das Dauerimplantat zur Embolisierung nach einem der Ansprüche 1 bis 7, worin der Kopfteil gebildet wird unter der Einwirkung der Oberflächenspannung des Metalls, wenn die Schlinge an das distale Endteil des Metallbandrings geschweißt wird.

9. Ein Verfahren zur Herstellung eines Dauerimplantats zur Embolisierung nach Anspruch 8, umfassend den Schritt des Einführens der Schlinge in den distalen Endbereich des Bandrings in Richtung des Rumpfes und Ausbilden des Kopfteils über eine Metalloberflächenhaftung, wenn die Schlinge verschweißt wird.

## Revendications

1. Implant à demeure (10A) pour l'embolisation comprenant une bobine (11) composée d'un métal et s'étendant d'une partie-tête qui est sensiblement arrondie dans une manière semi-sphérique (12), située à une partie terminale distale de la bobine, jusqu'à une partie proximale de la bobine, dans lequel une boucle fermée en métal (14) est fournie à l'intérieure de ladite bobine pour qu'elle dépasse à partir de ladite partie-tête en direction de la partie terminale proximale de la bobine, la boucle étant soudée à la partie-tête semi-sphérique ; et
un élément contrôlant l'extension axiale (20), composé d'au moins un matériau de fil qui est plus mince que le matériau de fil métal formant ladite boucle, est fourni à l'intérieur de ladite boucle dans une telle manière que l'élément contrôlant l'extension axiale s'étend en direction bobine axiale de ladite bobine et passe à travers de ladite boucle, et les deux bouts du matériau de fil formant l'élément contrôlant l'extension axiale sont fixés dans une manière directe ou indirecte à la partie terminale proximale.

2. Implant à demeure selon la revendication 1, dans lequel l'élément contrôlant l'extension axiale et la boucle sont composés du même matériau métal que la bobine.

3. Implant à demeure selon la revendication 1 ou 2, dans lequel la bobine est composée du platine ou d'un alliage de platine.

4. Implant à demeure selon l'une quelconque des revendications 1 à 3, dans lequel l'élément contrôlant l'extension axiale est composé d'un matériau de fil ayant un diamètre de 20 µm ou moins.

5. Implant à demeure pour l'embolisation selon l'une quelconque des revendications 1 à 4, dans lequel l'élément contrôlant l'extension axiale est composé d'un fil tordu obtenu par la torsion d'une pluralité de matériaux de fil métal.

6. Implant à demeure pour l'embolisation selon l'une quelconque des revendications 1 à 5, dans lequel l'élément contrôlant l'extension axiale est tordu encore plus âpres l'insertion à travers la boucle.

7. Implant à demeure pour l'embolisation selon l'une quelconque des revendications 1 à 6, dans lequel la bobine est en outre formée pour avoir une forme secondaire.

8. Implant à demeure pour l'embolisation selon l'une quelconque des revendications 1 à 7, dans lequel la partie tête est formée sous l'effet de la tension superficielle du metal au moment de forger la boucle jusqu'à la partie terminale distale de la bobine en metal.

9. Methode de fabrication l'implant à demeure pour l'embolisation selon la revendication 8, comprenant les étapes de l'insertion la boucle dans la partie terminale distale de la boucle en direction proximale et la formation de la partie tête par une tension superficielle d'un métal au moment de forger la boucle.
